# EUROPÄISCHE PATENTSCHRIFT

(11) **EP 0 724 574 B1**
(45) Veröffentlichungstag und Bekanntmachung des Hinweises auf die Patenterteilung: **27.02.2002**
(21) Anmeldenummer: 94925431.2
(22) Anmeldetag: 04.08.1994
(51) Int. Cl.: C07D 239/52, C07D 239/34, C07D 239/46, A01N 43/54

(54) **3-METHOXY-2-PHENYL-ACRYLSÄUREMETHYLESTER**
3-METHOXY-2-PHENYLACRYLIC ACID METHYL ESTERS
METHYLESTERS D'ACIDE 3-METHOXY-2-PHENYLACRYLIQUE

(30) Priorität: 17.08.1993 DE 4327596
(43) Veröffentlichungstag der Anmeldung: 07.08.1996
(73) Patentinhaber: BAYER AG, 51368 Leverkusen (DE)
(72) Erfinder: GERDES, Peter, D-52080 Aachen (DE); GAYER, Herbert, D-40789 Monheim (DE); DEHNE, Heinz-Wilhelm, D-53125 Bonn (DE); DUTZMANN, Stefan, D-40721 Hilden (DE); WACHENDORFF-NEUMANN, Ulrike, D-40789 Monheim (DE)
(86) Internationale Anmeldenummer: EP9402587
(87) Internationale Veröffentlichungsnummer: WO9505367

(56) Entgegenhaltungen:
- EP-A- 0 468 695

## Beschreibung

Die Erfindung betrifft neue 3-Methoxy-2-phenyl-acrylsäuremethylester, ein Verfahren zu ihrer Herstellung und ihre Verwendung als Schädlingsbekämpfungsmittel.

Es ist bekannt, daß bestimmte 3-Methoxy-acrylsäuremethylester, wie beispielsweise die Verbindung 3-Methoxy-2-(6-phenyl-2-pyridylthio)-acrylsäuremethylester oder die Verbindung 3-Methoxy-2-[N-(6-phenyl-2-pyridyl)-N-methyl-amino]-acrylsäuremethylester oder die Verbindung 3-Methoxy-2-[5-(4-chlorphenyl)-3-pyridyloxy]-acrylsäuremethylester fungizide Eigenschaften besitzen (vergl. z.B. EP 383 117).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue 3-Methoxy-2-phenyl-acrylsäuremethylester der allgemeinen Formel (I), 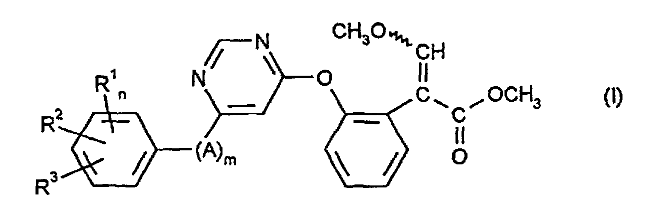 in welcher
- R¹: für Fluor, Chlor, Brom, Cyano, Nitro, Formyl, Carbamoyl, Thiocarbamoyl, Methyl, Ethyl, n-oder i-Propyl, n-, i-, s-oder t-Butyl, Methoxy, Ethoxy, n-oder i-Propoxy, n-, i-, s- oder t-Butoxy, Methylthio, Ethylthio, Methylsulfinyl, Methylsulfonyl, Allyl, Butenyl, Allyloxy, Butenyloxy, Trifluormethyl, Trifluormethoxy, Difluormethoxy, Trifluormethylthio, Difluorchlormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Cyclopropyl, Cyclopentyl, Cyclohexyl, 1-Pyrrolidinyl, 1-Piperidinyl, 1-Perhydroazepinyl, 4-Morpholinyl oder für jeweils gegebenenfalls ein- bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy steht,
- R²: entweder für Wasserstoff, Fluor, Chlor, Brom, Cyano, Nitro, Formyl, Carbamoyl, Thiocarbamoyl, Methyl, Ethyl, n-oder i-Propyl, n-, i-, s-oder t-Butyl, Methoxy, Ethoxy, n-oder i-Propoxy, n-, i-, s-oder t-Butoxy, Methylthio, Ethylthio, Methylsulfinyl, Methylsulfonyl, Allyl, Butenyl, Allyloxy, Butenyloxy, Trifluormethyl, Trifluormethoxy, Difluormethoxy, Trifluormethylthio, Difluorchlormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Cyclopropyl, Cyclopentyl, Cyclohexyl, 1-Pyrrolidinyl, 1-Piperidinyl, 1-Perhydroazepinyl, 4-Morpholinyl oder für jeweils gegebenenfalls ein-bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy steht und
- R³: für jeweils geradkettiges oder verzweigtes Halogenalkoxy oder Halogenalkylthio mit jeweils 1 bis 3 Kohlenstoffatomen und 1 bis 7 gleichen oder verschiedenen Halogenatomen - insbesondere Fluor, Chlor und/oder Brom - steht oder für eine -OCN-Guppe steht oder
- R² und R³: gemeinsam für einen gegebenenfalls einfach bis vierfach, gleich oder verschieden durch Fluor, Chlor, Brom, Methyl, Ethyl und/oder Trifluormethyl substituierten zweifach verknüpften Alkandiylrest mit 1 bis 3 Kohlenstoffatomen stehen, der 1 bis 2 Sauerstoff- oder Schwefelatome enthält,
- A: für Sauerstoff, steht,
- m: für eine Zahl 1 steht und
- n: für eine Zahl 0, 1, 2, 3 oder 4 steht.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden daß man die neuen 3-Methoxy-2-phenyl-acrylsäuremethylester der allgemeinen Formel (I), 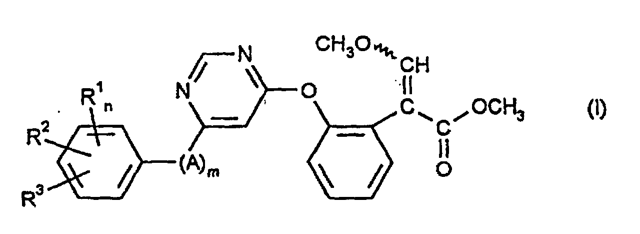 in welcher
- R¹: für Fluor, Chlor, Brom, Cyano, Nitro, Formyl, Carbamoyl, Thiocarbamoyl, Methyl, Ethyl, n-oder i-Propyl, n-, i-, s-oder t-Butyl, Methoxy, Ethoxy, n-oder i-Propoxy, n-, i-, s- oder t-Butoxy, Methylthio, Ethylthio, Methylsulfinyl, Methylsulfonyl, Allyl, Butenyl, Allyloxy, Butenyloxy, Trifluormethyl, Trifluormethoxy, Difluormethoxy, Trifluormethylthio, Difluorchlormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Cyclopropyl, Cyclopentyl, Cyclohexyl, 1-Pyrrolidinyl, 1-Piperidinyl, 1-Perhydroazepinyl, 4-Morpholinyl oder für jeweils gegebenenfalls ein- bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy steht,
- R²: entweder für Wasserstoff, Fluor, Chlor, Brom, Cyano, Nitro, Formyl, Carbamoyl, Thiocarbamoyl, Methyl, Ethyl, n-oder i-Propyl, n-, i-, s-oder t-Butyl, Methoxy, Ethoxy, n-oder i-Propoxy, n-, i-, s-oder t-Butoxy, Methylthio, Ethylthio, Methylsulfinyl, Methylsulfonyl, Allyl, Butenyl, Allyloxy, Butenyloxy, Trifluormethyl, Trifluormethoxy, Difluormethoxy, Trifluormethylthio, Difluorchlormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Cyclopropyl, Cyclopentyl, Cyclohexyl, 1-Pyrrolidinyl, 1-Piperidinyl, 1-Perhydroazepinyl, 4-Morpholinyl oder für jeweils gegebenenfalls ein-bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy steht und
- R³: für jeweils geradkettiges oder verzweigtes Halogenalkoxy oder Halogenalkylthio mit jeweils 1 bis 3 Kohlenstoffatomen und 1 bis 7 gleichen oder verschiedenen Halogenatomen - insbesondere Fluor, Chlor und/oder Brom - steht oder für eine -OCN-Guppe steht oder
- R² und R³: gemeinsam für einen gegebenenfalls einfach bis vierfach, gleich oder verschieden durch Fluor, Chlor, Brom, Methyl, Ethyl und/oder Trifluormethyl substituierten zweifach verknüpften Alkandiylrest mit 1 bis 3 Kohlenstoffatomen stehen, der 1 bis 2 Sauerstoff- oder Schwefelatome enthält,
- A: für Sauerstoff, Schwefel oder für eine Gruppierung der Formel -CH₂-O-; -O-CH₂-; -CH₂-S-, -S-CH₂- oder -C≡C- steht,
- m: für eine Zahl 0 oder 1 steht und
- n: für eine Zahl 0, 1, 2, 3 oder 4 steht,
erhält, wenn man 3-Methoxy-2-[2-(6-chlor-4-pyrimidinyloxy)-phenyl]-acrylsäure-methylester der Formel (II), 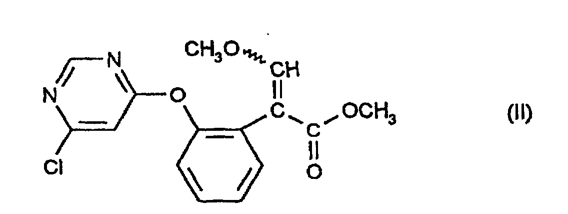 mit Verbindungen der Formel (III), 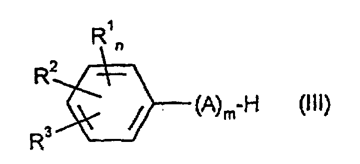 in welcher
R¹, R², R³, A, m und n die oben angegebene Bedeutung haben,
gegebenenfalls in Gegenwart eines Verdünnungsmittels und gegebenenfalls in Gegenwart eines Reaktionshilfsmittels sowie gegebenenfalls in Gegenwart eines Katalysators umsetzt.

Schließlich wurde gefunden, daß die neuen 3-Methoxy-2-phenyl-acrylsäuremethylester der allgemeinen Formel (I) gute Wirksamkeit gegenüber Schädlingen besitzen.

Überraschenderweise zeigen die erfindungsgemäßen 3-Methoxy-2-phenyl-acrylsäuremethylester der allgemeinen Formel (I) eine erheblich bessere Wirksamkeit gegenüber pflanzenschädigenden Mikroorganismen im Vergleich zu den aus dem Stand der Technik bekannten 3-Methoxy-acrylsäuremethylestern, wie beispielsweise die Verbindung 3-Methoxy-2-(6-phenyl-2-pyridylthio)-acrylsäuremethylester oder die Verbindung 3-Methoxy-2-[N-(6-phenyl-2-pyridyl)-N-methyl-amino]-acrylsäuremethylester oder die Verbindung 3-Methoxy-2-[5-(4-chlorphenyl)-3-pyridyloxy]-acrylsäuremethylester, welches chemisch und/oder wirkungsmäßig naheliegende Verbindungen sind.

Die erfindungsgemäßen 3-Methoxy-2-phenyl-acrylsäuremethylester sind durch die Formel (I) allgemein definiert.

Von besonderem Interesse sind Verbindungen der Formel (I), bei welchen
- R²: entweder für Wasserstoff, Fluor, Chlor, Brom, Cyano, Nitro, Formyl, Carbamoyl, Thiocarbamoyl, Methyl, Ethyl, n-oder i-Propyl, n-, i-, s-oder t-Butyl, Methoxy, Ethoxy, n-oder i-Propoxy, n-, i-, s-oder t-Butoxy, Methylthio, Ethylthio, Methylsulfinyl, Methylsulfonyl, Allyl, Butenyl, Allyloxy, Butenyloxy, Trifluormethyl, Trifluormethoxy, Difluormethoxy, Trifluormethylthio, Difluorchlormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Cyclopropyl, Cyclopentyl, Cyclohexyl, 1-Pyrrolidinyl, 1-Piperidinyl, 1-Perhydroazepinyl, 4-Morpholinyl oder für jeweils gegebenenfalls ein- bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy steht und
- R³: für Trifluor-, Difluor-, Fluor-, Difluorchlormethyl oder -methylthio, Pentafluor-, Tetrafluor-, Trifluor, Difluor-, Fluor-, Tetrafluorchlor-, Trifluorchlor-, Trifluordichlor-, Difluortrichlor-, Difluordichlor-, Difluorchlor-, Fluortetrachlor-, fluortrichlor, Fluordichlor-, Fluorchlor-ethoxy oder -ethylthio steht, oder für eine -OCN-Guppe steht oder
- R² und R³: gemeinsam für einen gegebenenfalls einfach bis vierfach, gleich oder verschieden durch Fluor, Chlor, Brom, Methyl, Ethyl und/oder Trifluormethyl substituierten zweifach verknüpften Alkandiylrest mit 1 bis 2 Kohlenstoffatomen stehen, der 1 bis 2 Sauerstoff- oder Schwefelatome enthält,
- A: für Sauerstoff steht,
- m: für die Zahl 1 steht und
- n: für die Zahl 0 steht;
wobei die Reste R² und R³ vorzugsweise in 4- und 2-Stellung angeordnet sind.

Im einzelnen seien außer den bei den Herstellungsbeispielen genannten Verbindungen die folgenden Verbindungen der allgemeinen Formel (I) genannt:

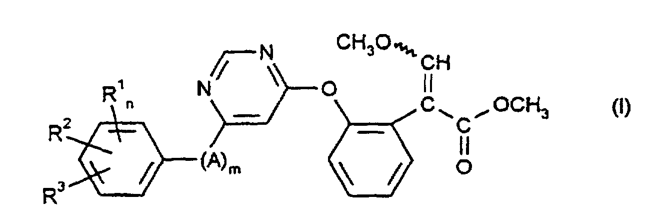

| R¹ₙ | R² | R³ | A | m |
|---|---|---|---|---|
| H | 2-CH₃ | 4-OCF₃ | -O- | 1 |
| H | 2-CH₃ | 4-O-CF₂-CF₂Cl | -O- | 1 |
| H | 2-CH₃ | 4-O-CF₂-CHF₂ | -O- | 1 |
| H | 2-Cl | 4-OCF₃ | -O- | 1 |
| H | H | 2-O-CHF₂ | -O- | 1 |
| H | 2-CH₃ | 4-OCF₃ | -O- | 1 |
| H | 2-CH₃ | 4-O-CF₂-CF₂Cl | -O- | 1 |
| H | 2-CH₃ | 4-O-CF₂-CHF₂ | -O- | 1 |
| H | 3,4-O-CF₂-CF₂-O- | | | -O-1 |
| H | 3,4-CF₂-O-CF₂-O- | | | -O-1 |
| H | 3,4-CF₂-O-CF₂- | | | -O-1 |
| H | 3,4-O-CF₂-CH₂-O- | | | -O-1 |
| H | 3,4-O-CF₂-CFCl-O- | | | -O-1 |
| H | 3,4-O-CF₂-CHF-O- | | | -O-1 |
| H | 3,4-CF₂-CHF-O- | | | -O-1 |
| H | 3,4-O-CF₂-O- | | | -O-1 |
| H | 3,4-O-CF₂-CF₂-O- | | | -O-1 |
| H | 3,4-CF₂-O-CF₂-O- | | | -O-1 |
| H | 3,4-CF₂-O-CF₂- | | | -O-1 |
| H | 3,4-O-CF₂-CH₂-O- | | | -O-1 |
| H | 3,4-O-CF₂-CFCl-O- | | | -O-1 |
| H | 3,4-O-CF₂-CHF-O- | | | -O-1 |
| H | 3,4-CF₂-CHF-O- | | | -O-1 |
| H | 3,4-O-CF₂-O- | | | -O-1 |

Verwendet man beispielsweise 3-Methoxy-2-[2-(6-chlor-4-pyrimidinyloxy)-phenyl]-acrylsäuremethylester und 2-Difluormethoxyphenol als Ausgangsstoffe, so läßt sich der Reaktionsablauf des erfindungsgemäßen Verfahrens durch das folgende Formelschema darstellen:

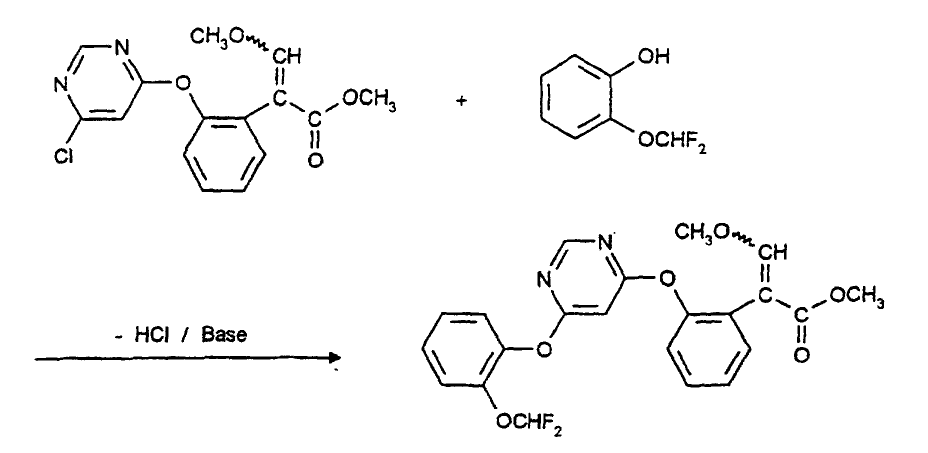

Der zur Durchführung des erfindungsgemäßen Verfahrens als Ausgangsverbindung benötigte 3-Methoxy-2-[2-(6-chlor-4-pyrimidinyloxy)-phenyl]-acrylsäuremethylester ist durch die Formel (II) definiert.
Der 3-Methoxy-2-[2-(6-chlor-4-pyrimidinyloxy)-phenyl]-acrylsäuremethylester der Formel (II) ist bekannt (vergl. z.B. EP 382 375).

Die zur Durchführung des erfindungsgemäßen Verfahrens weiterhin als Ausgangsstoffe benötigten Verbindungen sind durch die Formel (III) allgemein definiert. In dieser Formel (III) stehen R¹, R², R³, A und n vorzugsweise für diejenigen Reste und Indices, die bereits im Zusammenhang mit der Beschreibung der erfindungsgemäßen Verbindungen der Formel (I) als bevorzugt für diese Substituenten und diesen Index genannt wurden.
Die Verbindungen der Formel (III) sind allgemein bekannte Verbindungen der organischen Chemie.

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren wird außerdem gegebenenfalls in Gegenwart eines geeigneten Katalysators durchgeführt. Als solche kommen insbesondere Kupfer-(I)-Salze, wie beispielsweise Kupfer-(I)-chlorid infrage. Hierbei kann der Zusatz von katalytischen Mengen eines geeigneten Phasentransferkatalysators, wie beispielsweise 15-Krone-5, 18-Krone-6 oder Tris-[2-(2-methoxyethoxy)-ethyl]-amin von Vorteil sein.

Die Reaktionstemperaturen können bei der Durchführung des erfindungsgemäßen Verfahrens in einem größeren Bereich variiert werden. Im allgemeinen arbeitet man bei Temperaturen zwischen -20°C und +180°C, vorzugsweise bei Temperaturen zwischen 0°C und +150°C.

Das erfindungsgemäße Verfahren wird üblicherweise unter Normaldruck durchgeführt. Es ist jedoch auch möglich unter erhöhtem oder vermindertem Druck zu arbeiten.

Zur Durchführung des erfindungsgemäßen Verfahrens setzt man pro Mol an 3-Methoxy-2-[2-(6-chlor-4-pyrimidinyloxy)-phenyl]-acrylsäuremethylester der Formel (II) im allgemeinen 1,0 bis 3,0 Mol, vorzugsweise 1,0 bis 1,5 Mol an Verbindung der Formel (III) und gegebenenfalls 0,1 bis 3,0 Mol, vorzugsweise 0,5 bis 1,5 Mol an als Reaktionshilfsmittel verwendeter Base ein. Die Reaktionsdurchführung, Aufarbeitung und Isolierung der Reaktionsprodukte erfolgt jeweils in Analogie zu bekannten Verfahren (vergl. hierzu beispielsweise EP 382 375 sowie die Herstellungsbeispiele).

Die Reinigung der Endprodukte der Formel (I) erfolgt mit Hilfe üblicher Verfahren, beispielsweise durch Säulenchromatographie oder durch Umkristallisieren.
Die Charakterisierung erfolgt mit Hilfe des Schmelzpunktes oder bei nicht kristallisierenden Verbindungen mit Hilfe des Brechungsindex oder der Protonen-Kernresonanzspektroskopie (¹H-NMR).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Krankheiten im Obst- und Gemüseanbau, wie beispielsweise gegen den Erreger der Tomatenbraunfäule (Phytophthora infestans) oder gegen den Erreger der falschen Rebenmehltaues (Plasmopara viticola) oder gegen den Erreger des Bohnengrauschimmels (Botrytis cinerea) oder gegen den Erreger des Apfelschorfes (Venturia inaequalis) oder zur Bekämpfung von Getreidekrankheiten wie beispielsweise gegen den Erreger der Halmbruchkrankheit des Weizens (Pseudocercosporella herpotrichoides) oder gegen den Erreger des echten Getreidemehltaues (Erysiphe graminis) oder gegen den Erreger der Netzfleckenkrankheit der Gerste (Pyrenophora teres) oder gegen den Erreger der Braunfleckenkrankheit der Gerste oder des Weizens (Cochliobolus sativus) oder gegen den Erreger der Braunspelzigkeit des Weizens (Leptosphaeria nodorum) oder gegen Fusariumarten oder zur Bekämpfung von Reiskrankheiten, wie beispielsweise gegen den Erreger der Reisfleckenkrankheit (Pyricularia oryzae) oder gegen den Erreger der Reisstengelkrankheit (Pellicularia sasakii) eingesetzt werden. Daneben besitzen die erfindungsgemäßen Wirkstoffe eine gute in vitro-Wirksamkeit.

Daneben eignen sich die Wirkstoffe zur Bekämpfung von tierischen Schädlingen, vorzugsweise Arthropoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam.

Zu den oben erwähnten Schädlingen gehören:
Aus der Ordnung der Isopoda z.B. Oniscus asellus, Armadillidium vulgare, Porcellio scaber;
aus der Ordnung der Diplopoda z.B. Blaniulus guttulatus;
aus der Ordnung der Chilopoda z.B. Geophilus carpophagus, Scutigera spec.;
aus der Ordnung der Symphyla z.B. Scutigerella immaculata;
aus der Ordnung der Thysanura z.B. Lepisma saccharina;
aus der Ordnung der Collembola z.B. Onychiurus armatus;
aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria; aus der Ordnung der Dermaptera z.B. Forficula auricularia;
aus der Ordnung der Isoptera z.B. Reticulitermes spp.;
aus der Ordnung der Anoplura z.B. Phylloxera vastatrix, Pemphigus spp., Pediculus humanus corporis, Haematopinus spp., Linognathus spp.;
aus der Ordnung der Mallophaga z.B. Trichodectes spp., Damalinea spp.;
aus der Ordnung der Thysanoptera z.B. Hercinothrips femoralis, Thrips tabaci;
aus der Ordnung der Heteroptera z.B. Eurigaster spp., Dysdercus intermedius, Piesma quadrata, Cimex lectularius, Rhodnius prolixus, Triatoma spp.;
aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.;
aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana;
aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica;
Aus der Ordnung der Hymenoptera z.B. Diprion spp., Hoplocampa spp., Lasius spp., Monomorium pharaonis, Vespa spp.;
aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa;
aus der Ordnung der Siphonaptera z.B. Xenopsylla cheopis. Ceratophyllus spp.;
aus der Ordnung der Arachnida z.B. Scorpio maurus, Latrodectus mactans;
aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich dabei durch eine hohe insektizide und akarizide Wirksamkeit aus.

Sie lassen sich mit besonders gutem Erfolg zur Bekämpfung von pflanzenschädigenden Insekten, wie beispielsweise gegen die grüne Pfirsichblatlaus (Mycus persicae) oder gegen die Larven der Kohlschabe (Plutella xylostella) oder gegen die Larven des Heerwurms (Spodoptera frugiperda) oder gegen die gemeine Spinnmilbe (Tetranychus urticae) einsetzen.

Die Wirkstoffe können in Abhängigkeit von ihren jeweiligen physikalischen und/oder chemischen Eigenschaften in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infragen: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zinn verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

In vielen Fällen erhält man dabei synergistische Effekte, d.h. die Wirksamkeit der Mischung ist größer als die Wirksamkeit der Einzelkomponenten.

### Für die Mischungen kommen beispielsweise infrage:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyanophenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel dimethyldithiocarbamat, Nitrothal-isopropyl, Nuarimol,
Ofurace, Oxadixyl, Oxamocarb, Oxycarboxin,

Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chloretoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, ,Primiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die Wirkstoffe können bei der Anwendung als Fungizide als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen bei der Anwendung als Fungizide in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden bei der Anwendung als Fungizide im allgemeinen Wirkstoffmengen von 0,001 bis 50 g je Kilogramm Saatgut, vorzugsweise 0,01 bis 10 g benötigt.

Bei der Behandlung des Bodens sind bei der Anwendung als Fungizide Wirkstoffkonzentrationen von 0,00001 bis 0,1 Gew.-%, vorzugsweise von 0,0001 bis 0,02 Gew.-% am Wirkungsort erforderlich.

Die erfindungsgemäßen Wirkstoffe können bei der Anwendung als Insektizide und Akarizide ebenfalls in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden hählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können bei der Anwendung als Insektizide und Akarizide ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetze Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gewichtsprozent Wirkstoff, vorzugsweise zwischen 0,0001 und 1 Gewichtsprozent liegen.

Die Anwendung geschieht in einer den Anwendungsformen angepaßten üblichen Weise.

Die Herstellung und die Verwendung der erfindungsgemäßen Wirkstoffe geht aus den nachfolgenden Beispielen hervor.

### Herstellungsbeispiele:

### Beispiel 1:

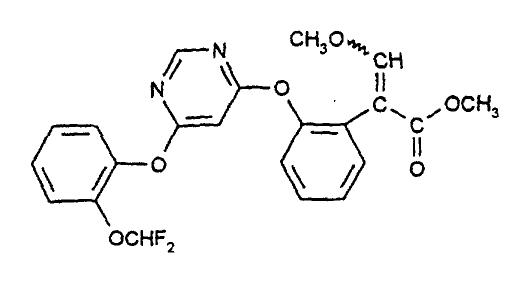

Zu einer Mischung von 0,8 g (0,0025 Mol) (E)-3-Methoxy-2-[2-(6-chlor-4-pyrimidinyloxy)-phenyl]-acrylsäuremethylester (vergl. z.B. EP 382 375) und 0,4 g (0,0025 Mol) 2-Difluormethoxyphenol (vergl. z.B. J. Fluorine Chem., 59, 257-67 [1992]; EP 166 287) in 50 ml absolutem N,N-Dimethylformamid gibt man bei Raumtemperatur unter Rühren 0,7 g (0,005 Mol) Kaliumcarbonat und 0,1 g Kupfer-(I)-chlorid, erhitzt den Reaktionsansatz anschließend langsam auf Siedetemperatur und rührt 4 Stunden bei dieser Temperatur. Zur Aufarbeitung gibt man zu der abgekühlten Reaktionsmischung 50 ml Wasser zu und extrahiert zweimal mit jeweils 30 ml Essigester. Die vereinigten organischen Phasen werden getrocknet, im Vakuum eingeengt und der Rückstand durch Chromatographie an Kieselgel (Laufmittel: Dichlormethan/Essigester 10:1) gereinigt.

Man erhält 0,9 g (82 % der Theorie) an (E)-3-Methoxy-2-{2-[6-(2-Difluormethoxy-phenoxy)-4-pyrimidinyloxy]-phenyl}-acrylsäuremethylester als Öl vom Brechungsindex = 1,5569.

In entsprechender Weise und gemäß den allgemeinen Angaben zur Herstellung erhält man die folgenden 3-Methoxy-2-phenyl-acrylsäuremethylester der allgemeinen Formel (I):

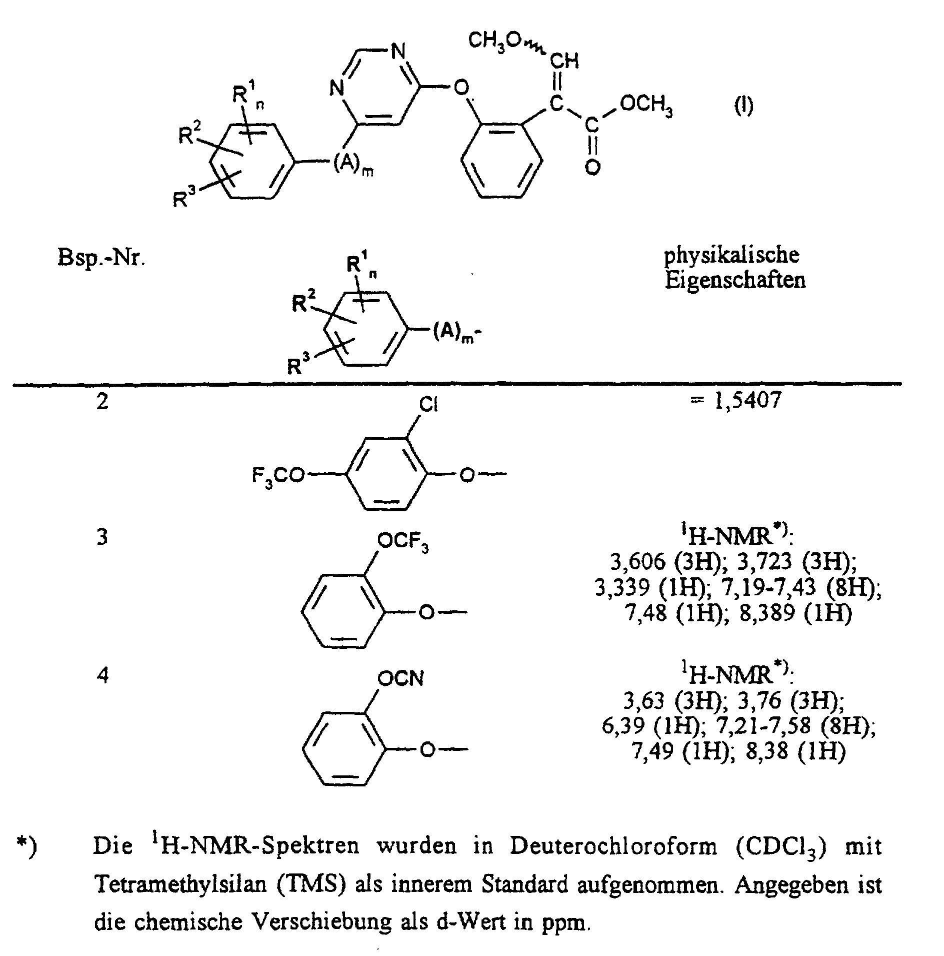

### Anwendungsbeispiele:

In den folgenden Anwendungsbeispielen wurden die nachstehend aufgeführten Verbindungen als Vergleichssubstanzen eingesetzt: 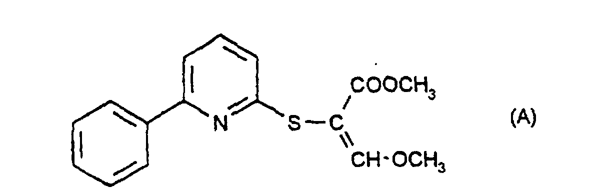 3-Methoxy-2-(6-phenyl-2-pyridylthio)-acrylsäuremethylester 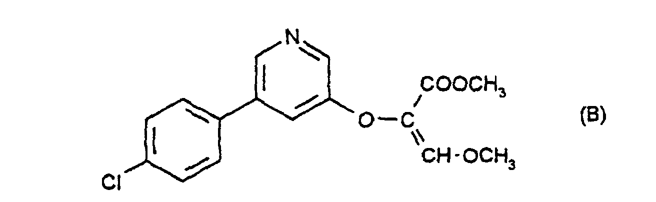 3-Methoxy-2-[5-(4-chlorphenyl)-3-pyridyloxy]-acrylsäuremethylester 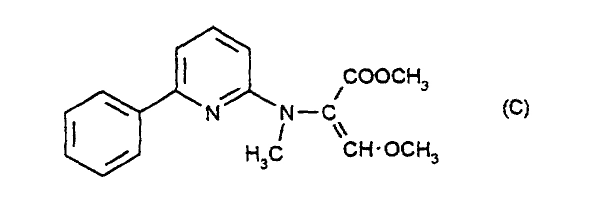 3-Methoxy-2-[N-(6-phenyl-2-pyridyl)-N-methyl-amino]-acrylsäuremethylester (alle bekannt aus EP 383 117)

### Beispiel A:

### Phytophthora-Test (Tomate) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und Emulgator und verdünnt das Könzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Sporensuspension von Phytophthora infestans inokuliert.

Die Pflanzen werden dann in einer Inkubationskabine bei 20°C und einer relativen Luftfeuchtigkeit von ca. 100 % aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen 1 und 2, die bei einer Wirkstoffkonzentration von 10 ppm einen mehr als 50 %igen Wirkungsgrad zeigen.

### Beispiel B:

### Plasmopara-Test (Rebe) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Sporensuspension von Plasmopara viticola inokuliert und verbleiben dann einen Tag in einer Feuchtkammer bei 20°C bis 22°C und 100% relativer Luftfeuchtigkeit. Anschließend werden die Pflanzen 5 Tage im Gewächshaus bei 21°C und 90% Luftfeuchtigkeit aufgestellt. Die Pflanzen werden dann angefeuchtet und einen Tag in eine Feuchtkammer gestellt.

6 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen 3 und 4, die bei einer Wirkstoffkonzentration von 10 ppm einen mehr als 70 %igen Wirkungsgrad zeigen.

### Beispiel C:

### Venturia-Test (Apfel) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Konidiensuspension des Apfelschorferregers (Venturia inaequalis) inokuliert und verbleiben dann einen Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigt in diesem Test z.B. die Verbindung gemäß dem Herstellungsbeispiel 4, die bei einer Wirkstoffkonzentration von 10 ppm einen mehr als 90 %igen Wirkungsgrad zeigt.

### Beispiel D:

### Botrytis-Test (Buschbohne) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt zwei kleine mit Botrytis cinerea bewachsene Agarstückchen aufgelegt. Die inokulierten Pflanzen werden in einer abgedunkelten feuchten Kammer bei 20°C aufgestellt.

3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen gemäß den Herstellungsbeispielen 1 und 2, die bei einer Wirkstoffkonzentration von 10 ppm einen mehr als 80 %igen Wirkungsgrad zeigt.

### Beispiel E:

### Pseudocercosporella herpotrichoides-Test (Weizen) / protektiv

- Lösungsmittel:: 10 Gewichtsteile N-Methylpyrrolidon
- Emulgator:: 0,6 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca. 10°C und einer relativen Luftfeuchtigkeit von ca. 80 % aufgestellt.

21 Tage nach der Inokulation erfolgt die Auswertung.

Eine deutliche Überlegenheit in der Wirksamkeit gegenüber dem Stand der Technik zeigt in diesem Test z.B. die Verbindung gemäß Herstellungsbeispiel 1, welche bei einer Wirkstoffkonzentration von 400 g/ha mehr als 70 Wirkungsgrade zeigt.

### Beispiel F:

### Erysiphe-Test (Gerste) / protektiv

- Lösungsmittel:: 10 Gewichtsteile N-Methylpyrrolidon
- Emulgator:: 0,6 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen 1, 2 und 3, die bei einer Wirkstoffkonzentration von 200 g/ha einen mehr als 80 %igen Wirkungsgrad zeigen.

### Beispiel G:

### Leptosphaeria nodorum-Test (Weizen) / protektiv

- Lösungsmittel:: 10 Gewichtsteile N-Methylpyrrolidon
- Emulgator:: 0,6 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigt bei diesem Test z.B. die Verbindung gemäß dem Herstellungsbeispiel 1, welches bei einer Wirkstoffkonzentration von 40 g/ha einen mehr als 80 %igen Wirkungsgrad zeigt.

### Beispiel H:

### Cochliobolus sativus-Test (Gerste) / protektiv

- Lösungsmittel:: 10 Gewichtsteile N-Methylpyrrolidon
- Emulgator:: 0,6 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigt bei diesem Test z.B. die Verbindung gemäß dem Herstellungsbeispiel 1, welches bei einer Wirkstoffkonzentration von 40 g/ha einen mehr als 70 %igen Wirkungsgrad zeigt.

### Beispiel I:

### Pyrenophora teres-Test (Gerste) / protektiv

- Lösungsmittel:: 10 Gewichtsteile N-Methylpyrrolidon
- Emulgator:: 0,6 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigt bei diesem Test z.B. die Verbindung gemäß dem Herstellungsbeispiel 1, welches bei einer Wirkstoffkonzentration von 40 g/ha einen mehr als 80 %igen Wirkungsgrad zeigt.

### Beispiel J:

### Myzus-Test

- Lösungsmittel:: 31 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Keimlinge der dicken Bohne (Vicia faba), welche von der Pfirsichblattlaus (Myzus persicae) befallen sind, werden in die Wirkstoffzubereitung der gewünschten Konzentration getaucht und in eine Plastikdose gelegt.

Nach der gewünschten Zeit wird die Abtötung in Prozent bestimmt. Dabei bedeutet 100 %, daß alle Blattläuse abgetötet wurden; 0 % bedeutet, daß keine Blattläuse abgetötet wurden.

Eine deutliche Überlegenheit in der Wirksamkeit gegenüber dem Stand der Technik zeigt bei diesem Test z.B. die Verbindung gemäß dem Herstellungsbeispiel 2, welche bei einer Wirkstoffkonzentration von 0,1 % nach 9 Tagen einen 100 %igen Abtötungsgrad zeigt.

### Beispiel K:

### Plutella-Test

- Lösungsmittel:: 31 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Kohlblätter (Brassica oleracea) werden mit der Wirkstoffzubereitung der gewünschten Konzentration behandelt. Ein behandeltes Blat wird in eine Plastikdose gelegt und mit Larven (L2) der Kohlschabe (Plutella xylostella) besetzt. Nach 3 Tagen wird jeweils ein unbehandeltes Blat für die Nachfütterung verwendet.

Nach der gewünschten Zeit wird die Abtötung in Prozent bestimmt. Dabei bedeutet 100 %, daß alle Larven abgetötet wurden; 0 % bedeutet, daß keine Larven abgetötet wurden.

Eine deutliche Überlegenheit in der Wirksamkeit gegenüber dem Stand der Technik zeigt bei diesem Test z.B. die Verbindung gemäß dem Herstellungsbeispiel 2, welche bei einer Wirkstoffkonzentration von 0,1 % nach 6 Tagen einen 100 %igen Abtötungsgrad zeigt.

### Beispiel L:

### Spodoptera-Test

- Lösungsmittel:: 31 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Auf eine genormte Menge Kunstfutter wird eine angegebene Menge Wirkstoffzubereitung in der gewünschten Konzentration pipettiert. In sechsfacher Wiederholung werden je eine Larve (L3) des Heerwurms (Spodoptera frugiperda) auf das Futter gesetzt.

Nach der gewünschten Zeit wird die Abtötung in Prozent bestimmt. Dabei bedeutet 100 %, daß alle Larven abgetötet wurden; 0 % bedeutet, daß keine Larven abgetötet wurden.

Eine deutliche Überlegenheit in der Wirksamkeit gegenüber dem Stand der Technik zeigt bei diesem Test z.B. die Verbindung gemäß dem Herstellungsbeispiel 2, welche bei einer Wirkstoffkonzentration von 0,1 % nach 6 Tagen einen 100 %igen Abtötungsgrad zeigt.

### Beispiel M:

### Tetranychus-Test (OP-resistent)

- Lösungsmittel:: 31 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Entwicklungsstadien der gemeinen Spinnmilbe (Tetranychus urticae) befallen sind, werden in eine Wirkstoffzubereitung der gewünschten Konzentration getaucht.

Nach der gewünschten Zeit wird die Wirkung in Prozent bestimmt. Dabei bedeutet 100 %, daß alle Spinnmilben abgetötet wurden; 0 % bedeutet, daß keine Spinnmilben abgetötet wurden.

Eine deutliche Überlegenheit in der Wirksamkeit gegenüber dem Stand der Technik zeigt bei diesem Test z.B. die Verbindung gemäß dem Herstellungsbeispiel 2, welche bei einer Wirkstoffkonzentration von 0,1 % nach 14 Tagen einen 100 %igen Abtötungsgrad zeigt.

## Patentansprüche

1. Verbindungen der allgemeinen Formel (I), 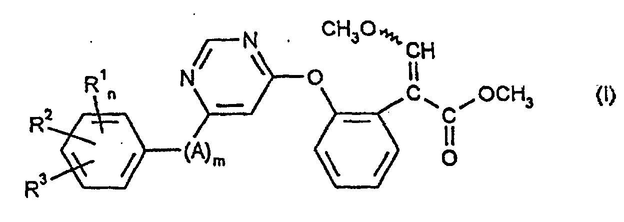 in welcher
R¹ für Fluor, Chlor, Brom, Cyano, Nitro, Formyl, Carbamoyl, Thiocarbamoyl, Methyl, Ethyl, n-oder i-Propyl, n-, i-, s-oder t-Butyl, Methoxy, Ethoxy, n-oder i-Propoxy, n-, i-, s- oder t-Butoxy, Methylthio, Ethylthio, Methylsulfinyl, Methylsulfonyl, Allyl, Butenyl, Allyloxy, Butenyloxy, Trifluormethyl, Trifluormethoxy, Difluormethoxy, Trifluormethylthio, Difluorchlormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Cyclopropyl, Cyclopentyl, Cyclohexyl, 1-Pyrrolidinyl, 1-Piperidinyl, 1-Perhydroazepinyl, 4-Morpholinyl oder für jeweils gegebenenfalls ein- bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy steht,
R² entweder für Wasserstoff, Fluor, Chlor, Brom, Cyano, Nitro, Formyl, Carbamoyl, Thiocarbamoyl, Methyl, Ethyl, n-oder i-Propyl, n-, i-, s-oder t-Butyl, Methoxy, Ethoxy, n-oder i-Propoxy, n-, i-, s-oder t-Butoxy, Methylthio, Ethylthio, Methylsulfinyl, Methylsulfonyl, Allyl, Butenyl, Allyloxy, Butenyloxy, Trifluormethyl, Trifluormethoxy, Difluormethoxy, Trifluormethylthio, Difluorchlormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Cyclopropyl, Cyclopentyl, Cyclohexyl, 1-Pyrrolidinyl, 1-Piperidinyl, 1-Perhydroazepinyl, 4-Morpholinyl oder für jeweils gegebenenfalls ein-bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy steht und
R³ für jeweils geradkettiges oder verzweigtes Halogenalkoxy oder Halogenalkylthio mit jeweils 1 bis 3 Kohlenstoffatomen und 1 bis 7 gleichen oder verschiedenen Halogenatomen - insbesondere Fluor, Chlor und/oder Brom - steht oder für eine -OCN-Guppe steht oder
R² und R³ gemeinsam für einen gegebenenfalls einfach bis vierfach, gleich oder verschieden durch Fluor, Chlor, Brom, Methyl, Ethyl und/oder Trifluormethyl substituierten zweifach verknüpften Alkandiylrest mit 1 bis 3 Kohlenstoffatomen stehen, der 1 bis 2 Sauerstoff- oder Schwefelatome enthält, und
A für Sauerstoff,
m für eine Zahl 1 steht und
n für eine Zahl 0, 1, 2, 3 oder 4 steht.

2. Schädlingsbekämpfungsmittel, **gekennzeichnet durch** einen Gehalt an mindestens einer Verbindung der Formel (I) nach Anspruch 1.

3. Verfahren zur Bekämpfung von Pflanzenschädlingen, **dadurch gekennzeichnet, daß** man Verbindungen der Formel (I) nach Anspruch 1 auf Schädlinge und/oder ihren Lebensraum einwirken läßt.

4. Verfahren zur Herstellung von 3-Methoxy-2-phenyl-acrylsäuremethylestern der allgemeinen Formel (I),
wie in Anspruch 1 definiert,
**dadurch gekennzeichnet, daß** man 3-Methoxy-2-[2-(6-chlor-4-pyrimidinyloxy)-phenyl]-acrylsäuremethylester der Formel (II), 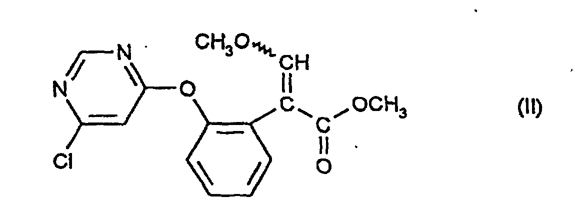 mit Verbindungen der Formel (III), 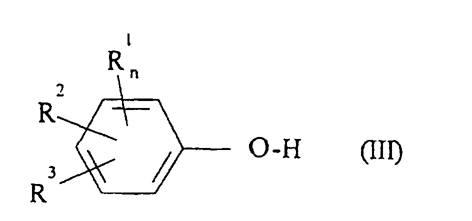 in welcher
R¹, R², R³, und n die in Anspruch 1 angegebene Bedeutung haben,
gegebenenfalls in Gegenwart eines Verdünnungsmittels und gegebenenfalls in Gegenwart eines Reaktionshilfsmittels sowie gegebenenfalls in Gegenwart eines Katalysators umsetzt.

5. Verwendung von Verbindungen der Formel (I) nach den Ansprüchen 1 bis 4 zur Bekämpfung von Pflanzenschädlingen.

6. Verfahren zur Herstellung von Schädlingsbekämpfungsmitteln, **dadurch gekennzeichnet, daß** man Verbindungen der Formel (I) nach den Ansprüchen 1 bis 4 mit Streckmitteln und/oder oberflächenaktiven Mitteln vermischt.

## Claims

1. Compounds of the general formula (I) 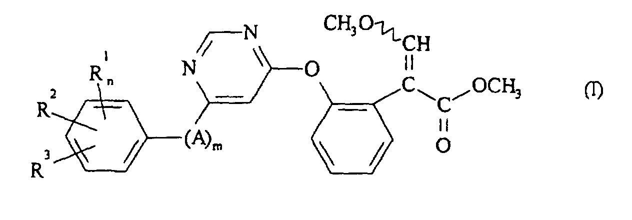 in which
R¹ represents fluorine, chlorine, bromine, cyano, nitro, formyl, carbamoyl, thiocarbamoyl, methyl, ethyl, n- or i-propyl, n-, i-, s- or t-butyl, methoxy, ethoxy, n- or i-propoxy, n-, i-, s- or t-butoxy, methylthio, ethylthio, methylsulphinyl, methylsulphonyl, allyl, butenyl, allyloxy, butenyloxy, trifluoromethyl, trifluoromethoxy, difluoromethoxy, trifluoromethylthio, difluorochloromethylthio, trifluoromethylsulphinyl, trifluoromethylsulphonyl, dimethylamino, diethylamino, acetyl, acetoxy, methylsulphonyloxy, ethylsulphonyloxy, methoxycarbonyl, ethoxycarbonyl, hydroximinomethyl, hydroximinoethyl, methoximinomethyl, methoximinoethyl, ethoximinomethyl, ethoximinoethyl, cyclopropyl, cyclopentyl, cyclohexyl, 1-pyrrolidinyl, 1-piperidinyl, 1-perhydroazepinyl, 4-morpholinyl, or phenyl, phenoxy, benzyl or benzyloxy, each of which is optionally monosubstituted to trisubstituted by identical or different substituents from the series comprising fluorine, chlorine, bromine, methyl, ethyl, methoxy, ethoxy, trifluoromethyl and/or trifluoromethoxy,
R² either represents hydrogen, fluorine, chlorine, bromine, cyano, nitro, formyl, carbamoyl, thiocarbamoyl, methyl, ethyl, n- or i-propyl, n-, i-, s- or t-butyl, methoxy, ethoxy, n- or i-propoxy, n-, i-, s- or t-butoxy, methylthio, ethylthio, methylsulphinyl, methylsulphonyl, allyl, butenyl, allyloxy, butenyloxy, trifluoromethyl, trifluoromethoxy, difluoromethoxy, trifluoromethylthio, difluorochloromethylthio, trifluoromethylsulphinyl, trifluoromethylsulphonyl, dimethylamino, diethylamino, acetyl, acetoxy, methylsulphonyloxy, ethylsulphonyloxy, methoxycarbonyl, ethoxycarbonyl, hydroximinomethyl, hydroximinoethyl, methoximinomethyl, methoximinoethyl, ethoximinomethyl, ethoximinoethyl, cyclopropyl, cyclopentyl cyclohexyl, 1-pyrrolidinyl, 1-piperidinyl, 1-perhydroazepinyl, 4-morpholinyl, or phenyl, phenoxy, benzyl or benzyloxy, each of which is optionally monosubstituted to trisubstituted by identical or different substituents from the series comprising fluorine, chlorine, bromine, methyl, ethyl, methoxy, ethoxy, trifluoromethyl and/or trifluoromethoxy, and
R³ represents in each case straight-chain or branched halogenoalkoxy or halogenoalkylthio, each of which has 1 to 3 carbon atoms and 1 to 7 identical or different halogen atoms - in particular fluorine, chlorine and/or bromine -, or an -OCN group, or
R² and R³ together represent a divalent alkanediyl radical which has 1 to 3 carbon atoms, contains 1 to 2 oxygen or sulphur atoms and is optionally monosubstituted to tetrasubstituted by identical or different substituents from the series comprising fluorine, chlorine, bromine, methyl, ethyl and/or trifluoromethyl, and
A represents oxygen,
m represents a number 1, and
n represents a number 0, 1, 2, 3 or 4.

2. Pesticides, **characterized in that** they contain at least one compound of the formula (I) according to Claim 1.

3. Process for combating plant pests, **characterized in that** compounds of the formula (I) according to Claim 1 are allowed to act on pests and/or their habitat.

4. Process for the preparation of methyl 3-methoxy-2-phenyl-acrylates of the general formula I as defined in Claim 1,
**characterized in that** methyl 3-methoxy-2-[2-(6-chloro-4-pyrimidinyloxy)-phenyl]-acrylate of the formula (II) 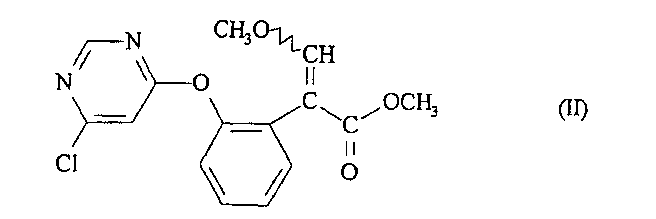 is reacted with compounds of the formula (III), 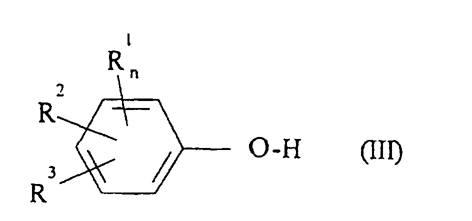 in which
R¹, R², R³ and n have the meaning given in Claim 1,
if appropriate in the presence of a diluent and if appropriate in the presence of a reaction auxiliary and if appropriate in the presence of a catalyst.

5. Use of compounds of the formula (I) according to Claims 1 to 4 for combating plant pests.

6. Process for the preparation of pesticides, **characterized in that** compounds of the formula (I) according to Claims 1 to 4 are mixed with extenders and/or surface-active agents.

## Revendications

1. Composés de formule générale (I) 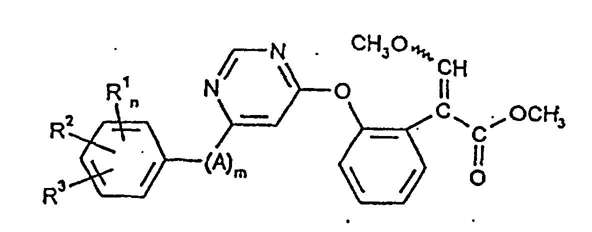 dans laquelle :
R¹ représente le fluor, le chlore, le brome, le groupe cyano, nitro, formyle, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, méthylsulfinyle, méthylsulfonyle, allyle, butényle, allyloxy, butényloxy, triflorométhyle, trifluorométhoxy, difluorométhoxy, trifluorométhylthio, difluorochlorométhylthio, trifluorométhylsulfinyle, trifluorométhylsulfonyle, diméthylamino, diéthylamino, acétyle, acétoxy, méthylsulfonyloxy, éthylsulfonyloxy, méthoxycarbonyle, éthoxycarbonyle, hydroximinométhyle, hydroximino-éthyle, méthoximinométhyle, méthoximinoéthyle, éthoximinométhyle, éthoximino-éthyle, cyclopropyle, cyclopentyle, cyclohexyle, 1-pyrrolidinyle, 1-pipéridinyle, 1-perhydro-azépinyle, 4-morpholinyle ou un groupe phényle, phénoxy, benzyle ou benzyloxy portant éventuellement dans chaque cas un à trois substituants, identiques ou différents, fluoro, chloro, bromo, méthyle, éthyle, méthoxy, éthoxy, trifluorométhyle et/ou trifluorométhoxy,
R² représente l'hydrogène, le fluor, le chlore, le brome, le groupe cyano, nitro, formyle, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertio-butoxy, méthylthio, éthylthio, méthylsulfinyle, méthylsulfonyle, allyle, butényle, allyloxy, butényloxy, trifluorométhyle, trifluorométhoxy, difluorométhoxy, trifluorométhylthio, difluorochlorométhylthio, trifluorométhylsulfinyle, trifluorométhylsulfonyle, diméthylamino, diéthylamino, acétyle, acétoxy, méthylsulfonyloxy, éthylsulfonyloxy, méthoxycarbonyle, éthoxycarbonyle, hydroximinométhyle, hydroximino-éthyle, méthoximinométhyle, méthoximino-éthyle, éthoximinométhyle, éthoximino-éthyle, cyclopropyle, cyclopentyle, cyclohexyle, 1-pyrrolidinyle, 1-pipéridinyle, 1-perhydro-azépinyle, 4-morpholinyle, ou bien un groupe phényle, phénoxy, benzyle ou benzyloxy portant éventuellement dans chaque cas un à trois substituants, identiques ou différents, fluoro, chloro, bromo, méthyle, éthyle, méthoxy, éthoxy, trifluorométhyle et/ou trifluorométhoxy, et
R³ représente un groupe halogénalkoxy ou halogénalkylthio linéaire ou ramifié ayant chacun 1 à 3 atomes de carbone et 1 à 7 atomes d'halogènes identiques ou différents - en particulier fluor, chlore et/ou brome - ou un groupe -OCN ou bien
R² et R³ forment conjointement un reste alcanediyle ayant 1 à 3 atomes de carbone, ayant deux liaisons, portant éventuellement un à quatre substituants fluoro, chloro, bromo, méthyle, éthyle et/ou trifluorométhyle identiques ou différents et contenant 1 ou 2 atomes d'oxygène ou de soufre,
A représente l'oxygène,
m représente le nombre 1 et
n représente le nombre 0, 1, 2, 3 ou 4.

2. Compositions pesticides, **caractérisées par** une teneur en au moins un composé de formule (I) suivant la revendication 1.

3. Procédé pour combattre des parasites des plantes, **caractérisé en ce qu'**on fait agir des composés de formule (I) suivant la revendication 1 sur les parasites et/ou sur leur milieu.

4. Procédé de production d'esters méthyliques d'acides 3-méthoxy-2-phényl-acryliques de formule générale (I) telle que définie dans la revendication 1,
**caractérisé en ce qu'**on fait réagir des esters méthyliques d'acide 3-méthoxy-2-[2-(6-chloro-4-pyrimidinyloxy)-phényl]-acrylique de formule (II) 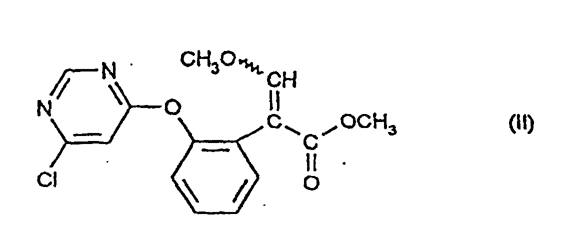 avec des composés de formule (III) 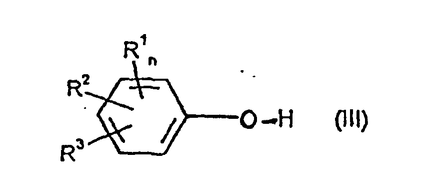 dans laquelle
R¹, R², R³ et n ont la définition indiquée dans la revendication 1,
éventuellement en présence d'un diluant et en présence éventuelle d'un auxiliaire de réaction de même que, le cas échéant, en présence d'un catalyseur.

5. Utilisation de composés de formule (I) suivant les revendications 1 à 4 pour combattre des parasites des plantes.

6. Procédé de préparation de compositions pesticides, **caractérisé en ce qu'**on mélange des composés de formule (I) suivant les revendications 1 à 4 avec des diluants et/ou des agents tensio-actifs.
